# EUROPEAN PATENT SPECIFICATION

(11) **EP 2 117 463 B1**
(45) Date of publication and mention of the grant of the patent: **14.11.2018**
(21) Application number: 08726090.7
(22) Date of filing: 26.02.2008
(51) Int. Cl.: A61F 2/06, A61F 2/07, A61F 2/88, A61F 2/90

(54) **RADIOPAQUE POLYMERIC STENT**
RÖNTGENDICHTER POLYMERSTENT
ENDOPROTHÈSE POLYMÈRE RADIO-OPAQUE

(30) Priority: 07.03.2007 US 905460 P
(43) Date of publication of application: 18.11.2009
(73) Proprietor: Boston Scientific Limited, Hamilton HM11 (BM)
(72) Inventor: HEADLEY, F., Anthony, Jr., Playmouth, MN 55447 (US); CLERC, Claude, Marlborough, MA 01752 (US); DAMARATI, John, Marlborough, MA 01752 (US); WHITCHER, Forrest, D., Allston, MA 02134 (US)
(74) Representative: Vossius & Partner Patentanwälte Rechtsanwälte mbB
(86) International application number: PCT/US2008/002507
(87) International publication number: WO 2008/112076

(56) References cited:
- EP-A2- 0 804 909
- WO-A1-2006/081448
- WO-A2-00/44309
- WO-A2-2004/110502
- WO-A2-2006/053270
- US-A- 5 674 242
- US-A1- 2006 004 440
- US-A1- 2006 142 840
- US-A1- 2006 163 773
- US-A1- 2007 038 290
- US-B1- 6 860 901
- US-B1- 7 018 401

## Description

### FIELD OF THE INVENTION

The present invention generally relates to an implantable stent, and more particularly, to radiopaque polymeric stents and methods for making the same.

### BACKGROUND OF THE INVENTION

Implantable stents are devices that are placed in a body structure, such as a blood vessel or body cavity, to provide support and to maintain the structure open. Generally, implantable stents made from metallic or polymeric wires or strands comprise a flexible tubular body composed of one or more rigid but flexible filament elements. Wire or filament stents have been formed into braids, weaves or knits using techniques suitable for such construction. In some stents, the filaments extend in helix configuration with a center line of the tubular body about a common axis. In braided constructions, the filaments can be interlaced to form a tubular body having a symmetrical arrangement of filaments, e.g. where the number of filaments in each direction of a braid is divisible by two. Generally, the greater the diameter of the tubular body, the more filaments are used to impart stability to the body.

Generally, the proper deployment of the stent in a body cavity, such as in a blood vessel, the esophagus or other body cavity, requires a medical practitioner to follow movement of the stent through the body to the precise position at which the stent is to be deployed. To that end, radiopaque stents have been developed that allow the medical practitioner to track the position of the stent during movement through the body using fluoroscope and/or x-ray devices.

The opacity of a stent image tends to vary with the material and type of process used to create the stent. For example, radiopacity may be limited by the location of radiopaque materials in or on the stent. Furthermore, introducing radiopaque materials into stent filaments can produce undesirable mechanical alterations to filament mechanical properties. As such, a minimal amount of radiopaque material is typically used in creating radiopaque stents to prevent undesired alteration of the physical properties of the stent.
Creating a stent with a minimal amount of radiopaque material, however, reduces the practioner's ability to track the position of the stent during movement through the body. As such, there exists a need for an improved radiopaque stent that has greater radiopacity, yet maintains its overall functionality during and after various medical procedures.

US 2007/0038290 A1 discloses polymeric composite stents reinforced with fibers for implantation into a bodily lumen.

US 5,674,242 A discloses an endoprosthetic device for insertion at a vascular site. The device is composed of a structural member carrying a polymer member having an embedded therapeutic compound. The polymer member is formed of a shape-memory polymer for expansion upon exposed to a selected stimulus. The structural member is designed for coexpansion with the polymer member when the device is exposed to the stimulus.

US 7,018,401 B1 discloses a device comprising a plurality of shape memory wires woven together to form a body suitable for implantation into an anatomical structure, the body having first and second ends, the shape memory wires crossing each other to form a plurality of cells and a plurality of angles, at least one of the angles being obtuse, at least one of the cells being defined by only four sides, and both ends of at least one shape memory wire being located proximate one end of the body. The value of the at least one obtuse angle may be increased by axially compressing the body.

US 2006/0142840 A1 discloses an implantable prosthesis comprising a tubular graft comprising opposed open ends and having yarns in a textile pattern to define a textile wall having a luminal surface and an exterior surface. The implantable prosthesis further comprises a tubular, radially extensible member comprising a portion arranged in a closed zig-zag pattern, said pattern having a series of angled bends at proximal and distal ends thereof, and longitudinally extending members having opposed proximal and distal ends, said distal ends being disposed from the angled bends of said proximal end. The longitudinally extending members have a plurality of detents for securing said yarns within said textile pattern at one of said opposed open ends. The yarns of said textile patterns are securably disposed to said detents.

WO 2006/053270 A2 discloses an implantable stent including a plurality of elongate wires braided to form a hollow tubular structure having a tubular wall to define an interior surface and an exterior surface and having opposed open first and second ends, wherein the opposed open first and second ends are atraumatic ends. The wires at the first end are arranged in a series of closed loops with each loop having an apex defined by a bend in one of the wires and having an opposed base defined by crossing of adjacent wires, wherein the apex of adjacent closed loops are longitudinally offset from one another. The wires include composite wires to enhance visibility of the wires to provide improved external imaging of the wires in the body.

### SUMMARY OF THE INVENTION

The invention is defined by the features of the independent claims.

The invention relates to an implantable radiopaque stent adapted to be disposed in a body lumen. In one aspect of the invention, a plurality of radiopaque filaments are arranged for permanent attachment to a hollow tubular structure. The phrase arranged for permanent attachment" means that the radiopaque filaments are incorporated into the stent as a part of or all of the stent wall; for example, interweaving or braiding the filaments into a stent wall or interweaving or braiding the radiopaque filaments with other filaments to form the stent wall; or attaching or joining the radiopaque filaments to the stent by various means, such as by adheringly bonding it, or by looping it through the stent structure, or by mechanically fastening it to the stent structure. In some embodiments the radiopaque filaments are present along substantially the entire length of the stent. In other embodiments the radiopaque filaments are present along only one or more portions of the stent. In still other embodiments, the filaments may be selectively positioned along one or more portions of the stent. In some embodiments the radiopaque filaments are substantially, if not entirely, radiopaque along their length. In some embodiments, the radiopaque filaments are radiopaque at the selective portions along their length.

The terms "wire" and "filament" as used herein includes polymeric and metallic wires and filaments, as well as composites made of either or both classes of materials.

The filaments are arranged in a linear direction traverse to a longitudinal length of the structure, the structure having a tubular wall that defines an inner surface and an outer surface and opposing first open end and second open end. The radiopaque filaments improve external imaging of the tubular structure on fluoroscope or x-ray imaging equipment.

The stent of this aspect of the invention desirably may have a plurality of filaments arranged in a helix configuration about a centerline of the tubular structure with a common axis.

The stent of this aspect of the invention desirably may have the plurality of radiopaque filaments prepared by compounding a radiopaque powder with a polymeric material. Desirably, the radiopaque powder can be a metal, alloy, or ceramic, typically selected from the group consisting of gold, platinum, tungsten, platinum-tungsten, palladium, iridium, platinum-iridium, rhodium, tantalum or combinations thereof or barium sulfate, bismuth subcarbonate, bismuth oxychloride, bismuth trioxide. The radiopaque material may be encapsulated in another material and then incorporated into the filaments. Encapsulating the radiopaque material into another material may advantageously allow the radiopaque filaments to be formed easily and/or be less toxic.

Preferably, the polymeric material may be selected from polyester, polypropylene, polyethylene, polyurethane, polynaphthalene, polytetrafluoroethylene, expanded polytetrafluoroethylene, silicone, and combinations thereof.

The stent of this aspect of the invention desirably may have bioabsorbable and/or biodegradable material included in the radiopaque filament. The bioabsorbable and/or biodegradable materials may include poly-L-lactide, poly-D-lactide, polyglycolide, polydioxanone, polycaprolactone, and polygluconate, polylactic acid-polyethylene oxide copolymers, modified cellulose, collagen, poly(hydroxybutyrate), polyanhydride, polyphosphoester, poly(amino acids), poly (alpha-hydroxy acid) and combinations thereof.

The stent of this aspect of the present invention has filaments that terminate at the second end, wherein the filaments at the first end are arranged in a series of closed loops with each loop having an apex defined by a bend in one of the filaments and having an opposed base defined by crossing of adjacent filaments, and further wherein the apex of adjacent closed loops are longitudinally offset from one and the other.

The stent of this aspect of the present invention desirably may have filaments that are arranged in any known manner in the art including weaving, knitting, braiding, twisting, tying, laser or electron beam etched, mechanically etched, molded, injection molded, layer deposition, dipped and other techniques.

The stent of this aspect of the present invention may also be partially or fully coated with a polymeric material. The stent may further include a hollow tubular graft disposed partially or fully over the interior or the exterior surface. Desirably, the graft is a polymeric material. The polymeric material may be selected from polyester, polypropylene, polyethylene, polyurethane, polynaphthalene, polytetrafluoroethylene, expanded polytetrafluoroethylene, silicone, and combinations thereof.

The stents of the invention may optionally include a polymeric coating which contains radiopaque particles. For example, a polymeric coating, such as a silicone, may include radiopaque particles dispersed therein. Once coated onto the stent, the coating serves its purpose as a coating as well as a radiopaque marker. The polymeric coating may serve to fill the spaces or openings in the stent, and the entire device serve as a coated stent or stent-graft.

The stents of the invention may optionally include a polymeric covering that contains radiopaque particles. For example, the polymeric covering may cover the entire stent and be formed by dipping the stent in the polymeric material.

In another aspect of the invention, a plurality of elongate radiopaque filaments are braided together to form a hollow tubular structure having a tubular wall that defines an inner surface and an outer surface and opposing first open end and second open end. The tubular structure optionally includes a polymeric cover that may include radiopaque particles, wherein the radiopaque particles and the radiopaque filaments improve external imaging of the tubular structure on imaging equipment, such as fluoroscopic or x-ray equipment.

In one aspect of the invention the radiopaque filaments are made from a metallic or polymeric core having a polymeric radiopaque coating over the wire core. For example, the wire may be spray coated or dipped in the coating and incorporated into the stent structure. In another embodiment, the filaments are polymeric and have the radiopaque material incorporated within the polymer. For example, the polymeric composition may include a radiopaque material, with radiopaque filaments being formed from the composition by, for example, extrusion.

The stent of this aspect of the invention desirably may have the radiopaque filaments prepared by compounding a radiopaque powder with a polymeric material. Desirably, the radiopaque powder is a radiopaque material selected from gold, barium sulfate, ferritic particles, platinum, platinum-tungsten, palladium, platinum-iridium, rhodium, tantalum or combinations thereof, and the polymeric material is selected from the group consisting of polyester, polypropylene, polyethylene, polyurethane, polynaphthalene, polytetrafluoroethylene, expanded polytetrafluoroethylene, silicone, polyacrylate copolymers, and combinations thereof.
The stent of this aspect of the invention desirably may have bioabsorbable material included in the radiopaque filament. The bioabsorbable material may include poly-L-lactide, poly-D-lactide, polyglycolide, polydioxanone, polycaprolactone, and polygluconate, polylactic acid-polyethylene oxide copolymers, modified cellulose, collagen, poly(hydroxybutyrate), polyanhydride, polyphosphoester, poly(amino acids), poly (alpha-hydroxy acid) and combinations thereof.
In another aspect of the present invention, a method for making a radiopaque stent is provided. The method includes the steps of (i) providing radiopaque filaments, wherein the radiopaque filaments provide improved external imaging of the filaments in a body; and (ii) arranging the radiopaque filaments for permanent attachment to a hollow tubular structure in a linear direction traverse to a longitudinal length of the tubular structure, the tubular structure providing a tubular wall defining an interior surface and an exterior surface and having opposed open first and second ends.
The method of this aspect of the invention desirably may include preparing the radiopaque filament by compounding a radiopaque powder with a polymeric material. Desirably, the radiopaque powder includes a radiopaque material selected from gold, barium sulfate, ferritic particles, platinum, platinum-tungsten, palladium, platinum-iridium, rhodium, tantalum or combinations thereof.
The method of this aspect of the invention includes terminating the filaments at the second end, arranging the filaments at the first end in a series of closed loops with each loop having an apex defining a bend in the filaments and having an opposed base defined by crossing of adjacent filaments, and offsetting longitudinally the apex of adjacent closed loops from one and the other.

The method of this aspect of the invention desirably also may include arranging a plurality of polymeric radiopaque filaments in a helix configuration about a centerline of the tubular structure with a common axis, the plurality of polymeric radiopaque filaments arranged in a same linear direction.

The method of this aspect of the invention desirably may include preparing the polymeric radiopaque filaments by compounding a radiopaque powder with a polymeric material prior to extruding the filament. Desirably, the radiopaque powder includes a radiopaque material selected from gold, barium sulfate, ferritic particles, platinum, platinum-tungsten, palladium, platinum-iridium, rhodium, tantalum or combinations thereof.

The method of this aspect of the present invention desirably may include partially or fully coating or covering the stent with a polymeric material. The covering may be in the form of a partial or full cover or liner, such as a tubular structure which may be a conduit for liquid and/or prevent tissue ingrowth from encroaching on the stent lumen. Desirably, the covered stent or stent-graft is a polymeric material. The polymeric material may be selected from polyester, polypropylene, polyethylene, polyurethane, polynaphthalene, polytetrafluoroethylene, expanded polytetrafluoroe thylene, silicone, and combinations thereof.

The method desirably may include mixing a radiopaque powder in a silicone bath, such that, the coating includes radiopaque particles.

The stents and methods of the present invention may be used at strictures or damaged vessel sites. Such sites may suitably include bodily tissue, bodily organs, vascular lumens, non-vascular lumens and combinations thereof, such as, but not limited to, in the coronary or peripheral vasculature, esophagus, trachea, bronchi, colon, biliary tract, urinary tract, prostate, brain, stomach and the like.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the claims and equivalents thereof. The foregoing aspects and other attendant advantages of the present invention will become more readily appreciated by the detailed description taken in conjunction with the accompanying drawings.

### BRIEF DESCRIPTION OF THE DRAWINGS

FIG. 1 is a perspective view of a hollow, tubular stent according to the present invention.
FIG. 2 is an expanded view of a wall portion of the stent of FIG. 1 taken along the 2-2 axis showing a plurality of stent filaments.
FIG. 3 depicts a braided stent with a closed-end loop design having a plurality of welds at the closed end according to the present invention.
FIG. 4 depicts a thirty-six filament braided stent that includes radiopaque and non-radiopaque filaments.
FIGS. 5a-d illustrate a perpendicular view of the stent of FIG. 4 having four radiopaque filaments (2CW and 2 CCW), three radiopaque filaments, four radiopaque filaments and six radiopaque filaments, respectively.
FIGS. 6a-d illustrate a rotated 15 degree view of the stent of FIG. 4 having four radiopaque filaments (2CW and 2 CCW), three radiopaque filaments, four radiopaque filaments and six radiopaque filaments, respectively.
FIGS. 7a-d illustrate a rotated 30 degree view of the stent of FIG. 4 having four radiopaque filaments (2CW and 2 CCW), three radiopaque filaments, four radiopaque filaments and six radiopaque filaments, respectively.
FIGS. 8a-d illustrate a rotated 45 degree view of the stent of FIG. 4 having four radiopaque filaments (2CW and 2 CCW), three radiopaque filaments, four radiopaque filaments and six radiopaque filaments, respectively.
FIG. 9 depicts a stent having a covering of silicone according to the present invention.
FIG. 10 is a cross-sectional view of the stent of FIG. 8 showing an outer covering of silicone about the stent.
FIG. 11 is a cross-sectional view of the stent of FIG. 9 showing an inner covering of silicone about the stent.

Like reference symbols in the various drawings indicate like elements.

### DETAILED DESCRIPTION OF THE PREFERRED EMBODIMENTS

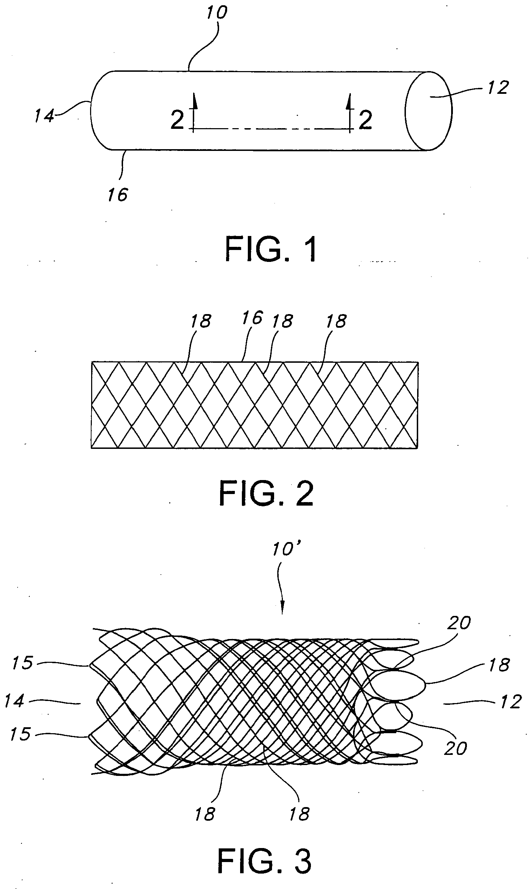

Referring now to FIG. 1, a stent 10 according to the present invention is disclosed. As shown in FIG. 1, the stent 10 includes a hollow tubular structure having opposed open ends 12, 14 and a tubular wall 16. A portion 2-2 of the tubular wall 16 is shown in FIG. 2 having a plurality of filaments or threads 18 which form the tubular wall 16. Tubular wall 16 is a distensible, open walled structure formed of filaments. The wall structure is radially expandable from a smaller radius to a larger radius. The radial expansion may occur as a result of the movement of filaments relative to one another or by plastic deformation of the filament material. The elongate filaments 18 traverse the length of the stent 10 in a direction traverse to the longitudinal length of the stent 10. The filaments 18 may be formed into the tubular wall 16 by braiding the filaments 18, winding the filaments 18, knitting the filaments 18, and combinations thereof. In some preferred embodiments, the filaments 18 are braided to form the tubular wall 16.

As used herein the term braiding and its variants refer to the diagonal intersection of elongate filaments, such as elongate wires, wire composites and polymeric filaments, so that each filament passes alternately over and under one or more of the other filaments, which is commonly referred to as an intersection repeat pattern. Useful braiding patterns include, but are not limited to, a diamond braid having a 1/1 intersection repeat pattern, a regular braid having a 2/2 intersection repeat pattern or a hercules braid having a 3/3 intersection repeat pattern. The passing of the filaments under and over one and the other results in slidable filament crossings that are not mechanically engaged or constrained.

Referring now to FIG. 3, in one preferred embodiment, the stent 10 is formed such that the elongate filaments 18 terminating at open end 12 may be mated and adjacently mated filaments may be secured to one and the other by welds 20 or by other suitable means. For example, in one preferred embodiment, the filaments 18 may be welded together through use of a welding material. In another preferred embodiment, the filaments 18 are heatingly and/or meltably fused together without the use of a welding material. In yet other preferred embodiments, for example, the filaments 18 are mechanically joined, such as, through the use of a small-sized or micro-fabricated clamp, crimpable tube, hypotube, and the like. Various techniques for welding filaments are known in the art.

The stent 10 shown in FIG. 3 is a braided stent that includes filaments 18 that are fully or partially composite filaments or wires 18. The filaments 18 provide improved external imaging of the stent in the body. Desirably, the enhanced visibility is enhanced radiopacity to provide improved fluoroscopic or x-ray visualization of the filaments in the body. Enhanced radiopacity may be achieved by using the below-described radiopaque materials in combination with a biocompatible and/or polymeric stent material. Such radiopaque materials are believed to be more visible under fluoroscopic or x-ray visualization due to their higher density than the corresponding biocompatible and/or polymeric stent material.

As shown in FIG. 3, in one preferred embodiment, the stent filaments 18 at the open end 14 may be bent to form closed loop ends 15 thereat. The loop ends 15 are substantially angular having approximately or about a 90° bend. The radius of curvature at the point of the bend is desirably minimized. In other words, the loop end 15 desirably has an angularly bent portion between substantially straight filament portions that do not otherwise have a portion with a significant radius of curvature. The loop ends 15, however, are not limited to angular bends of 90° and other bend angles may suitably be used. For example, angular bends with a bend angle from about 30° to about 150° are also useful. Other useful bend angles include from about 60° to about 120°, from about 70° to about 110°, from about 80° to about 100°, from about 85° to about 95°, and the like. The loop ends 15, however, are not limited to substantially angular bend-containing loops and other shaped loop ends, such as semi-circular, semi-elliptical and other smoothly curved or substantially smoothly curved loops, including but not limited to cathedral-shaped loops, may suitably be used.

The stent 10 depicted in FIG. 3 includes twenty-four filaments 18 of biocompatible material. In one preferred embodiment, the filaments 18 are relatively thin at a diameter of about 0.011 inches. The number of filaments and the diameters of the filaments, which may be the same or different, depicted in FIG. 3 are not limiting, and other numbers of filaments and other filament diameters may suitably be used. Desirably, an even number of filaments are used, for example from about 10 to about 36 wires.

The filaments 18 are made from a biocompatible material or biocompatible materials. Useful biocompatible materials include biocompatible metals, biocompatible alloys and biocompatible polymeric materials, including synthetic biocompatible polymeric materials and bioabsorbable or biodegradable polymeric materials. Desirably, the filaments 18 are biocompatible metals or alloys made from, but not limited to, nitinol, stainless steel, cobalt-based alloy such as Elgiloy, platinum, gold, titanium, tantalum, niobium, polymeric materials and combinations thereof. Useful synthetic biocompatible polymeric materials include, but are not limited to, polyesters, including polyethylene terephthalate (PET) polyesters, polypropylenes, polyethylenes, polyurethanes, polyolefins, polyvinyls, polymethylacetates, polyamides, naphthalane dicarboxylene derivatives, silks and polytetrafluoroethylenes. The polymeric materials may further include a metallic, a glass, ceramic or carbon constituent or fiber: Useful and nonlimiting examples of bioabsorbable or biodegradable polymeric materials include poly(L-lactide) (PLLA), poly(D,L-lactide) (PLA), poly(glycolide) (PGA), poly(L-lactide-coD,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polydioxanone (PDS), Polycaprolactone (PCL), polyhydroxybutyrate (PHBT), poly(phosphazene) poly(D,L-lactide-co-caprolactone) PLA/PCL), poly(glycolide-co-caprolactone) (PGA/PCL), poly(phosphate ester) and the like. In one preferred embodiment, for example, radiopaque materials such as barium sulfate and bismuth trioxide are compounded with the biocompatible material and are extruded into radiopaque filaments using a double extruder. Various radiopaque materials and their salts and derivatives may be used including, without limitation, bismuth, barium and its salts such as barium sulfate, tantalum, tungsten, gold, platinum and titanium, to name a few. Additional useful radiopaque materials may be found in U.S. Patent No. 6,626,936.

The filaments 18 made from polymeric materials also may include radiopaque materials, such as metallic-based powders or ceramic-based powders, particulates or pastes which may be incorporated into the polymeric material. The radiopaque material may be blended with the polymer composition from which the polymeric filament is formed, and subsequently fashioned into the stent. For example, in some preferred embodiments, a radiopaque powder is added to the polymeric material at extrusion time using a double screw extruder to form stent filaments. The radiopaque powder typically includes at least one element having a high atomic number such as bismuth, barium, tantalum, tungsten, gold, platinum.

For example, compounding approximately 50 to 70% weight of tantalum with polymeric material provides a filament comprising approximately 5 to 10% volume tantalum. Desirably, the low volume content of tantalum ensures that the filament maintains acceptable mechanical properties while being radiopaque.

In one preferred embodiment, the radiopaque filaments of the present invention include a longitudinal outer member concentrically disposed about a central core that extends along an axis of the outer member. Preferably, the outer member is formed of a metal, such as nitinol, that exhibits desirable properties, such as high elasticity and biocompatibility. The surface of the outer member may include a non-metal coating of, e.g., fluorocarbons, silicones, hydrophilic and lubricous biocompatible materials.) The central core of the radiopaque filaments includes a metal, such as tantalum, with a density greater than the longitudinal member to enhance the radiopacity of the filament and thus the stent from which it is formed. Preferably, the core is bonded to and substantially enclosed by the outer member such that the core does not have any substantial exposed surface and therefore does not contact body tissue when positioned within the body during use. In one preferred embodiment, the core is formed as a continuous solid member in intimate contact with and bonded to the interior portions of the outer member without the formation of substantial voids between the core and outer member. The core material preferably enhances the radiopacity of the filament but preferably does not substantially affect the mechanical performance of the filament.

In another preferred embodiment, the radiopaque filaments are formed as composite filaments including a central radiopaque core, an outer member, and an intermediate member between the core and the outer member. The intermediate member provides a barrier between the core and the outer member, and may be useful in composite filaments employing core and outer member materials that would be incompatible if contiguous, e.g. due to a tendency to form intermetallics.

In yet another preferred embodiment, the radiopaque filaments are formed as composite elements having a central radiopaque core, a structural outer member and a relatively thin annular outer cover layer. Suitable materials for the cover layer include tantalum, platinum, iridium, niobium, titanium and stainless steel.

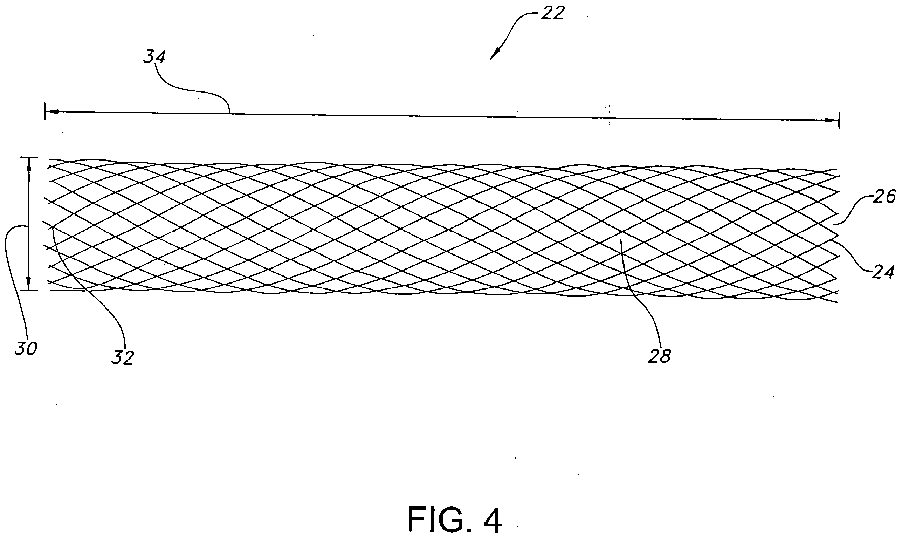

The radiopaque polymeric stent of the present invention may be formed in various designs. For example, in one preferred embodiment, the stent is a flexible self-expandable stent that includes inside and outside stent walls each fabricated by knitting memory alloy filaments into a net-like structure with a first filament zigzagged and a second filament zigzagged at a plurality of interlocked points with intersecting points there between. Advantageously, the configuration of the first and second filaments allows the stent walls to apply force against longitudinal contraction of the stent walls. Preferably, the interlocked points and the intersecting points form a plurality of diamond-shaped lattices in the structure of each stent wall. Preferably, the lattices are covered with radiopaque material. In one preferred embodiment, a tubing is fitted between the inside and outside stent walls, with each of the overlapped ends of the tubing and the stent walls being integrating into a single structure.
In another preferred embodiment, the radiopaque polymeric stent is formed from a single wire. The stent may be formed by either hand or machine weaving. The stent may be created by bending shape memory filaments around tabs projecting from a template, and weaving the ends of the filaments to create the body of the stent such that the filaments cross each other to form a plurality of angles. Preferably, at least one of the angles is formed obtuse. The value of the obtuse angle may be increased by axially compressing the stent structure.
The radiopaque polymeric stent of the present invention may be used with a valve assembly that may prevent undesirable matter from entering the stent. The valve assembly preferably includes first, second and third valve members that are extended from the central axis to an inner circumference wall of the first tubular structure and are spaced away from each other at an angle of approximately 120 degrees in a circumference direction of the first tubular structure. The first, second and third valve members may be provided with first, second and third passages, respectively, and a supporting valve member for connecting lower ends of the first, second and third valve members to an inner circumference wall of the first tubular structure.
Referring now to FIG. 4, an example 36-filament braided stent 22 having both radiopaque and non-radiopaque filaments is shown. The filaments are braided in a helix pattern of 18-filaments braided clock-wise (CW) 24 and 18-filaments braided counter-clockwise (CCW) 26. In one preferred embodiment, the filaments 24, 26 are about equally spaced 28 from one another. The helix configuration includes a diameter 30 of about 15 mm. At this diameter, the pitch of the stent is approximately 85 mm and the radial spacing 32 at the crossing of filaments 24, 26 is approximately 20[deg.] degrees. The length 34 of the stent 22 is about 85 mm.

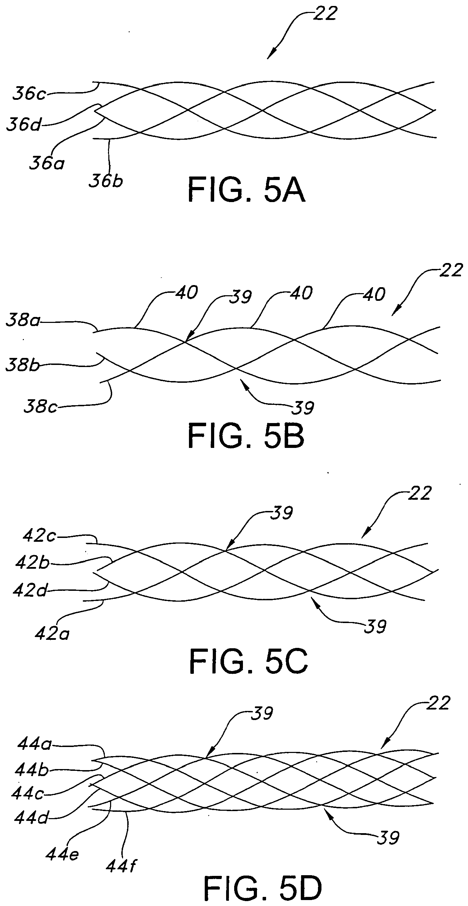

FIGS. 5a-d depict a perpendicular view of various arrangements of radiopaque filaments included in the stent 22 viewed under fluoroscope equipment. For example, FIG. 5a is an example not covered by the claims and illustrates a perpendicular view of four radiopaque filaments 36a, 36b, 36c, 36d attached to the stent. As shown in FIG. 5a, two radiopaque filaments 36a, 36b are arranged in a first linear direction 2CW (e.g., clock-wise) and the two radiopaque filaments 36c, 36d are arranged in a second linear direction 2CCW (e.g., counter clockwise) opposite the first linear direction. The four filaments 36a, 36b, 36c and 36d are spaced at approximately 90° degrees apart at their furthest points and cross at two points 180° degrees apart.

FIG. 5b depicts a perpendicular view of three radiopaque filaments 38a, 38b, 38c that are approximately equally spaced from one another and are arranged in a first linear direction. In this embodiment, the radiopaque filaments 38a, 38b, 38c are braided into the stent 22 at about 120° degrees apart. As shown in FIG. 5b, a void area 39 exists between the peaks 40 of the three radiopaque filaments 38a, 38b, and 38c. The void area 39 represents approximately twenty-five percent of the view.

FIG. 5c depicts a perpendicular view of four radiopaque filaments 42a, 42b, 42c and 42d that are all arranged in a first linear direction. In this embodiment, one radiopaque filament 42a is attached to the stent at about a 0° degree position. The third radiopaque filament 42c is attached to the stent at about a 180° degree position. In one preferred embodiment, the second and fourth radiopaque filaments 42b, 42d are attached to the stent 22 at about 120° degrees apart. In another preferred embodiment, the second and fourth radiopaque filaments 42b, 42d are attached to the stent 22 at about 100° and 280° degrees apart, respectively.

FIG. 5d depicts a perpendicular view of six radiopaque filaments 44a, 44b, 44c, 44d, 44e and 44f that are all arranged in a first linear direction and are attached to the stent 22 at approximately 60° degrees apart. As shown in FIGS. 5a and 5c, the radiopaque image of each pattern's radiopaque filaments appears similar and each stent's void area 39 is reduced to about 15% percent of the stent image. The radiopaque stent of FIG. 5d has only about a five percent void area 39.

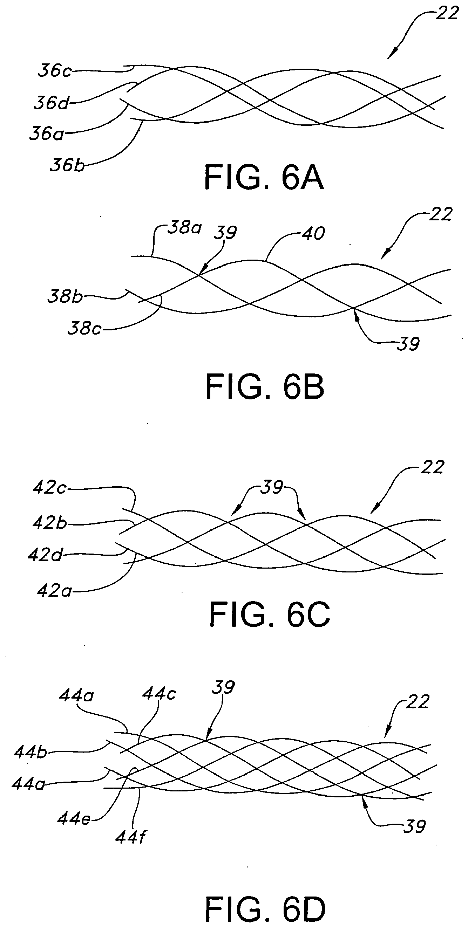

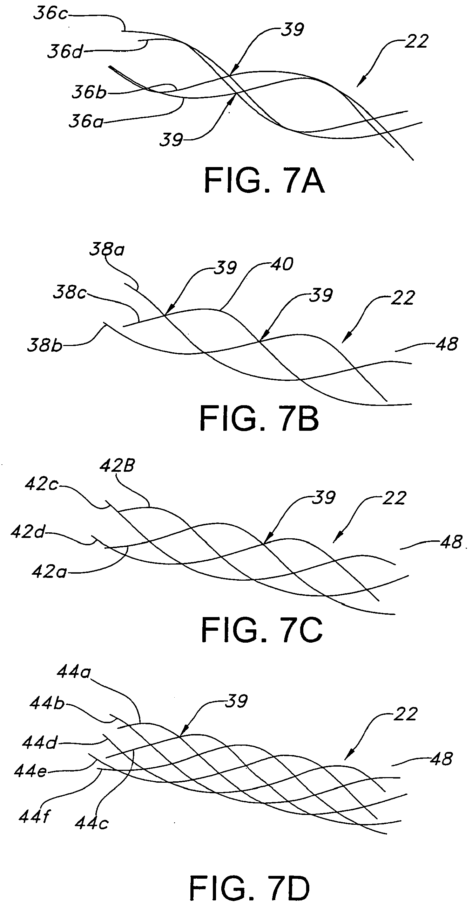

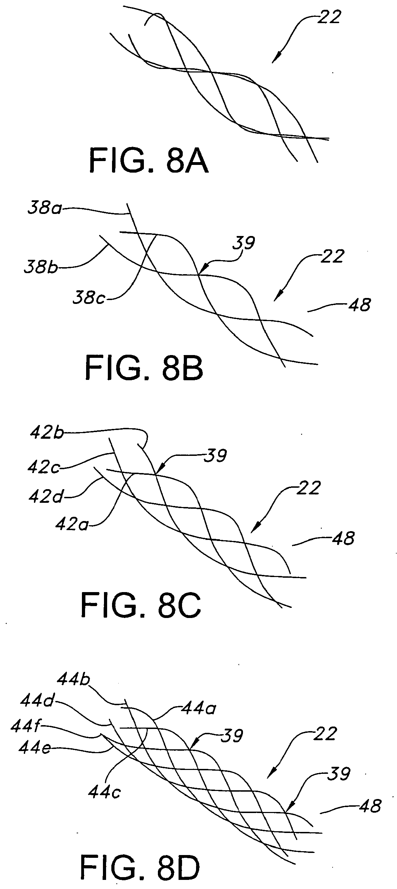

FIGS. 6a-d show the patterns of the radiopaque filaments of FIGS. 5a-d rotated at 15° degrees about two axes (Y-axis and Z-axis). FIGS. 7a-d and FIGS. 8a-d show the patterns of the radiopaque filaments of FIGS. 5a-d rotated at 30-degrees and 45-degrees about the same two axes, respectively.

As shown in FIG. 6a, the pattern image of radiopaque filaments of FIG. 5a distorts when the stent is viewed at a 15° degree angle. The image distortion in FIGS. 6b-d for the patterns shown in FIGS. 5b-d, respectively, when viewed at a 15° degree angle is minimal.

Referring now to FIG. 7a, when viewed at a 30° degree angle, the void area 39 of the radiopaque filament pattern of FIG. 5a increases to about 36% percent. The radiopaque patterns of FIGS 5b-d, when viewed at a 30° degree angle and depicted in FIGS. 7b-d, respectively, appear skewed with additional void areas 39 on one side 48 of the stent. As shown in FIGS. 7b-d, the amount of image distortion depends on the direction of the filament and the position from where the stent is viewed.

FIGS. 8a-d show the patterns of the radiopaque filaments of FIGS. 5a-d rotated at a 45° degree angle, respectively. As shown in FIGS. 8b-d, the void area 39 of the radiopaque filament patterns remain skewed with additional void areas 39 on one side 48 of the stent. Desirably, radiopaque filaments are arranged in the stent in the same direction (e.g., linear direction) to minimize distortion of the pattern when viewing the stent from angled perspectives.

Although FIGS 5a-8d depict various three, four, and six radiopaque filament patterns, the present invention is not limited to these embodiments. For example, in one preferred embodiment, a symmetrical pattern of 9-radiopaque filaments is arranged in a same linear direction in the stent resulting in about 99 percent of the stent being viewable from angled perspectives.

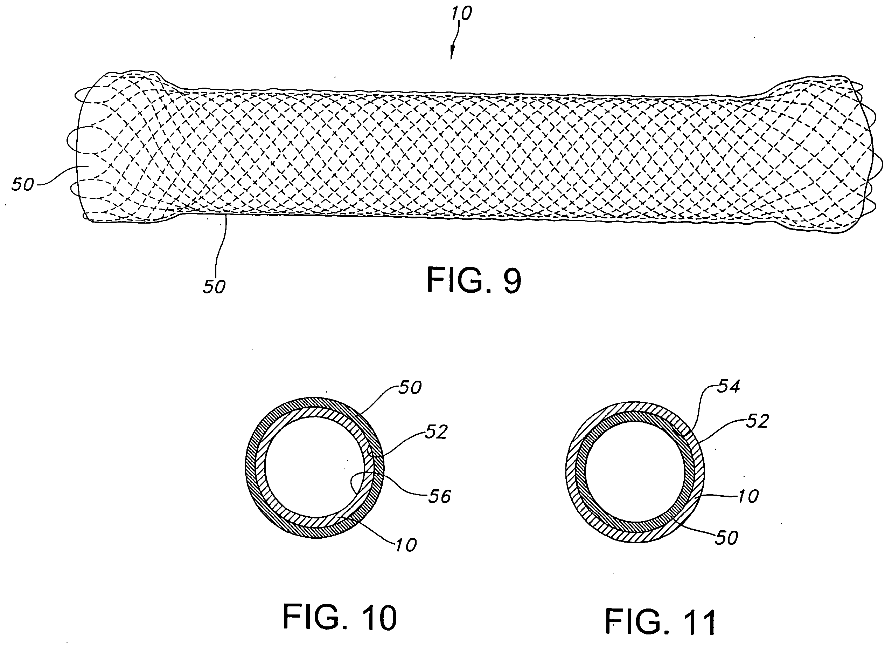

Referring now to FIG. 9, the stent 10 may be fully, substantially or partially covered or lined with a radiopaque polymeric material 50. The covering may be in the form of a tubular structure. Nonlimiting examples of polymeric coverings include silicone, polyurethane, polyethylene, polytetrafluoroetylene (PTFE) and expanded PTFE (ePTFE) and combnations and copolymers thereof. One nonlimiting example of a polymeric material is silicone. For example, in one preferred embodiment, the stent is covered with a silicon covering solution including radiopaque powder. In this preferred embodiment, radiopaque particles included in the powder are incorporated into the silicone covering providing improved radiopacity.

In another preferred embodiment, radiopaque material is added to the silicon covering solution by metallurgically alloying or by making clad composite structures. Radiopaque materials also may be filled into hollow cores, cavities or pores in the polymer matrix. Organic radiopaque powders containing elements or salts or oxides of elements such as bromine, iodine, iodide, barium, and bismuth also may be used instead of metal powders.

The radiopaque polymeric material 50 may be disposed on external surfaces 52 of the stent 10, as depicted in FIG. 10, or disposed on the internal surfaces 54 of the stent 10, as depicted in FIG. 11, or combinations thereof. The silicone covering may be suitably formed by dip coating the stent. The present invention is not limited to forming the silicone film by dip coating, and other techniques, such as spraying, may suitably be used. After applying the radiopaque silicone coating or film to the stent, the silicone may be cured. Desirably, the curing is low temperature curing, for example from about room temperature to about 90°C for a short period of time, for example from about 10 minutes or more to about 16 hours. The cured radiopaque silicone covering may also be sterilized by electronic beam radiation, gamma radiation ethylene oxide treatment and the like. Further details of the curing and/or sterilization techniques may be found in U.S. Patent Application No. 6,099,562,

Argon plasma treatment of the cured silicone may also be used.

With any embodiment of the stent 10, 22 of the present invention, the stent may be usable to maintain patency of a bodily vessel, such as in the coronary or peripheral vasculature, or non vascular lumens and ducts such as the esophagus, trachea, bronchi colon, small intestine, biliary tract, urinary tract, prostate, brain, and the like. Also, the stent 10,22 may be treated with any of the following: anti-thrombogenic agents (such as heparin, heparin derivatives, urokinase, and PPack (dextrophenylalanine proline arginine chloromethylketone); anti-proliferative agents (such as enoxaprin, angiopeptin, or monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-miotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, antithrombin compounds, platelet receptor antagonists, anti-thrombin antibodies, anti-platelet receptor antibodies, aspirin, prostaglandin inhibitors, platelet inhibitors and tick antiplatelet peptides); vascular cell growth promotors (such as growth factor inhibitors, growth factor receptor antagonists, transcriptional activators, and translational promotors); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.
In one aspect of the present invention, an implantable stent is provided. The stent includes a plurality of radiopaque filaments arranged for permanent attachment to a hollow tubular structure in a linear direction traverse to a longitudinal length of the hollow tubular structure, the tubular structure having a tubular wall that defines an inner surface and an outer surface and opposing first open end and second open end, the radiopaque filaments comprising a radiopaque material and a polymeric material. Preferably, the radiopaque filaments improve external imaging of the tubular structure on fluoroscope or x-ray imaging equipment.
The plurality of radiopaque filaments may be arranged in a helix configuration about a centerline of the tubular structure with a common axis. Preferably, the plurality of radiopaque filaments form the tubular structure.
The stent of this aspect of the present invention desirably may have a braided hollow tubular structure. Preferably, the stent of the present invention desirably is biodegradable.
The stent of this aspect of the present invention has the filaments terminate at the second end, wherein the filaments at the first end are arranged in a series of closed loops with each loop having an apex defined by a bend in the filaments and having an opposed base defined by crossing of adjacent filaments, and further wherein the apex of adjacent closed loops are longitudinally offset from one and the other.
The stent of this aspect of the present invention desirably may have the radiopaque material selected from the group consisting of gold, platinum, tungsten, platinum-tungsten, palladium, iridium, platinum-iridium, rhodium, tantalum, barium sulfate, bismuth subcarbonate, bismuth oxychloride, bismuth trioxide or combinations thereof. Desirably, the radiopaque material is a radiopaque powder.
The stent of this aspect of the present invention desirably may have the polymeric material selected from the group consisting of polyester, polypropylene, polyethylene, polyurethane, polynaphthalene, polytetrafluoroethylene, expanded polytetrafluoroethylene, silicone, and combinations thereof.
The stent of this aspect of the present invention desirably may have at least one radiopaque filament include a radiopaque material and a bioabsorbable material. Desirably, the bioabsorbable material is adapted to degrade in vivo. The bioabsorbable material may be selected from the group consisting of poly-L-lactide, poly-D-lactide, polyglycolide, polydioxanone, polycaprolactone, polygluconate, polylactic acid-polyethylene oxide copolymers, modified cellulose, collagen, poly(hydroxybutyrate), polyanhydride, polyphosphoester, poly(amino acids), poly (alpha-hydroxy acid) and combinations thereof.
Desirably, the radiopaque material is selected from the group consisting of gold, platinum, tungsten, platinum-tungsten, palladium, iridium, platinum-iridium, rhodium, tantalum, barium sulfate, bismuth subcarbonate, bismuth oxychloride, bismuth trioxide or combinations thereof.
The stent of this aspect of the present invention has the tubular structure covered with a polymeric material. Desirably, the polymeric material is selected from the group consisting of polyester, polypropylene, polyethylene, polyurethane, polynaphthalene, polytetrafluoroethylene, expanded polytetrafluoroethylene, silicone, and combinations thereof.
The stent of this aspect of the present invention has the polymeric material including radiopaque particles.
The stent of this aspect of the present invention further includes a polymeric covering. Desirably, the polymeric covering is biodegradable.
The stent of this aspect of the present invention has all of the radiopaque filaments arranged in a first linear direction.
In another aspect of the present invention, an implantable stent is provided that includes a plurality of elongate radiopaque filaments braided to form a hollow tubular structure having a tubular wall that defines an inner surface and an outer surface and opposing first open end and second open end. Desirably, the stent also includes a polymeric covering over the tubular structure.
The stent of this aspect of the present invention includes radiopaque material in the polymeric covering. Desirably, the polymeric covering is prepared by mixing a radiopaque powder with a polymeric material.
The stent of this aspect of the present invention preferably includes at least one of the plurality of radiopaque filaments having a radiopaque material and a biocompatible material. Desirably, the biocompatible material is selected from the group consisting of poly-L-lactide, poly-D-lactide, polyglycolide, polydioxanone, polycaprolactone, polygluconate, polylactic acid-polyethylene oxide copolymers, modified cellulose, collagen, poly(hydroxybutyrate), polyanhydride, polyphosphoester, poly(amino acids), poly (alpha-hydroxy acid) and combinations thereof. Desirably, the radiopaque material may be selected from the group consisting of gold, barium sulfate, ferritic particles, platinum, platinum-tungsten, palladium, platinum-iridium, rhodium, tantalum and combinations thereof.
The stent of this aspect of the present invention preferably includes the at least one of the plurality of radiopaque filaments having a radiopaque material and a polymeric material. Desirably, the radiopaque material is selected from the group consisting of gold, barium sulfate, ferritic particles, platinum, platinum-tungsten, palladium, platinum-iridium, rhodium, tantalum and combinations thereof. Preferably, the radiopaque material is a radiopaque powder.
The stent of this aspect of the present invention preferably includes selecting the polymeric material from the group consisting of polyester, polypropylene, polyethylene, polyurethane, polynaphthalene, polytetrafluoroethylene, expanded polytetrafluoroethylene, silicone, and combinations thereof.
The stent of this aspect of the present invention preferably may include at least one of the plurality of radiopaque filaments having a polymer or copolymer.
In yet another aspect of the present invention, a method for making an implantable stent includes providing a plurality of radiopaque filament, and arranging the at least one radiopaque filament for permanent attachment to a hollow tubular structure in a linear direction traverse to a longitudinal length of the tubular structure. The tubular structure provides a tubular wall defining an interior surface and an exterior surface and having opposed open first and second ends.
The method of this aspect of the invention includes providing a plurality of radiopaque filaments. Desirably, the method may also include arranging a plurality of radiopaque filament in a helix configuration about a centerline of the tubular structure with a common axis.
The method of this aspect of the present invention may include braiding a plurality of radiopaque filaments to form the tubular structure. Preferably, forming the at least one radiopaque filament comprises from a radiopaque material and a polymeric material.
The method of this aspect of the present invention may include selecting the polymeric material from the group consisting of polyester, polypropylene, polyethylene, polyurethane, polynaphthalene, polytetrafluoroethylene, expanded polytetrafluoroethylene, silicone, and combinations thereof. Desirably, the method may also include compounding the radiopaque material with the polymeric material. The radiopaque material may be a radiopaque powder.
The method of this aspect of the present invention may include selecting the radiopaque material from the group consisting of gold, platinum, tungsten, platinum-tungsten, palladium, iridium, platinum-iridium, rhodium, tantalum, barium sulfate, bismuth subcarbonate, bismuth oxychloride, bismuth trioxide or combinations thereof.
The method of this aspect of the present invention may further include forming the at least one radiopaque filament comprises from a radiopaque material and a biocompatible material. Desirably, the method also includes adapting the biocompatible material to degrade in vivo.
The method of this aspect of the present invention may include selecting the biocompatible material from the group consisting of poly-L-lactide, poly-D-lactide, polyglycolide, polydioxanone, polycaprolactone, polygluconate, polylactic acid-polyethylene oxide copolymers, modified cellulose, collagen, poly(hydroxybutyrate), polyanhydride, polyphosphoester, poly(amino acids), poly (alpha-hydroxy acid) and combinations thereof.
Desirably, the method of this aspect of the invention includes forming the at least one radiopaque filament from a polymer or copolymer.
The method of this aspect of the present invention includes forming a cover for the tubular structure by covering the tubular structure with a polymeric material. The method of this aspect of the invention may also include mixing a radiopaque powder in a silicon solution, such that, the cover includes radiopaque particles.
The method of this aspect of the present invention includes terminating the filaments at the second end, arranging the filaments at the first end in a series of closed loops with each loop having an apex defining a bend in the filaments and having an opposed base defined by crossing of adjacent filaments, and offsetting longitudinally the apex of adjacent closed loops from one and the other.
In yet another aspect of the present invention, a method for making an implantable stent includes braiding a plurality of elongate filaments to form a hollow tubular structure having a tubular wall that defines an inner surface and an outer surface and opposing first open end and second open end, and covering the tubular structure with a polymeric material including radiopaque particles, wherein the radiopaque particles improve external imaging of the tubular structure on fluoroscope or x-ray imaging equipment.

The method of this aspect of the present invention may include mixing a radiopaque powder with the polymeric material for covering the tubular structure. The method of this aspect of the present invention may also include forming the filaments by compounding a radiopaque material with a polymer material and/or biocompatible material..

Further, with any embodiment of the stent 10, 22, the general tubular shape may be varied. For example, the tubular shape may have a varied diameter, an inwardly flared end, an outwardly flared end and the like. Further, the ends of the stent may have a larger diameter than the middle regions of the stent. A braided stent with outwardly flared ends is further described in U.S. Patent No. 5,876,448.
The invention being thus described, it will now be evident to those skilled in the art that the same may be varied in many ways. Such variations are not to be regarded as a departure from the scope of the invention and all such modifications are intended to be included within the scope of the following claims.

## Claims

1. An implantable radiopaque stent (10; 22) comprising:
a plurality of radiopaque filaments (18; 24, 26; 36; 38; 42) arranged for permanent attachment to a hollow tubular structure in a linear direction traverse to a longitudinal length (34) of the hollow tubular structure, the tubular structure having a tubular wall that defines an inner surface and an outer surface and opposing first open end (14) and second open end (12), wherein the plurality of radiopaque filaments (18; 24, 26; 36; 38; 42) improves external imaging of the tubular structure on fluoroscope or x-ray imaging equipment,
wherein the tubular structure is covered with a polymeric material (50),
wherein the filaments (18; 24, 26; 36; 38; 42) terminate at the second end (12), and
wherein the filaments (18; 24, 26; 36; 38; 42) at the first end (14) are arranged in a series of closed loops (15) with each loop (15) having an apex defined by a bend in one of the filaments (18; 24, 26; 36; 38; 42) and having an opposed base defined by crossing of adjacent filaments, and further wherein the apices of adjacent closed loops (15) are longitudinally offset from one and the other,
**characterized in that**
the polymeric material (50) further includes radiopaque particles, the stent further comprises non-radiopaque filaments, and the radiopaque filaments are all arranged in the same linear direction.

2. The implantable radiopaque stent of claim 1, wherein the plurality of radiopaque filaments comprises a radiopaque material and a polymeric material, wherein the radiopaque material is a radiopaque powder.

3. The implantable radiopaque stent of claim 1, wherein the plurality of radiopaque filaments comprises a radiopaque material and a bioabsorbable material.

4. The implantable radiopaque stent of claim 1, wherein the polymeric covering is biodegradable.

5. A method for making an implantable stent (10; 22) comprising:
providing a plurality of filaments (18; 24, 26; 36; 38; 42), comprising both radiopaque and non- radiopaque filaments; and
arranging the plurality of filaments (18; 24, 26; 36; 38; 42) for permanent attachment to a hollow tubular structure in a linear direction traverse to a longitudinal length (34) of the tubular structure, wherein the radiopaque filaments are all arranged in the same linear direction, the tubular structure providing a tubular wall defining an interior surface and an exterior surface and having opposed open first (14) and second ends (12),
forming a cover for the tubular structure by covering the tubular structure with a polymeric material (50), wherein the polymeric material includes radiopaque particles,
terminating the filaments at the second end (12);
arranging the filaments at the first end (14) in a series of closed loops (15) with each loop (15) having an apex defining a bend in one of the filaments and having an opposed base defined by crossing of adjacent filaments; and
offsetting longitudinally the apices of adjacent closed loops (15) from one and the other.

6. The method of claim 5,
comprising forming the plurality of radiopaque filaments from a radiopaque material and a biocompatible material,
comprising forming the plurality of radiopaque filaments from a polymer or copolymer, further comprising mixing a radiopaque powder in a silicon bath such that the cover includes said radiopaque particles.

7. The implantable radiopaque stent of claim 2 or 3 or the method of claim 6, wherein the radiopaque material is selected from the group consisting of gold, platinum, tungsten, platinum-tungsten, palladium, iridium, platinum- iridium, rhodium, tantalum, barium sulfate, bismuth subcarbonate, bismuth oxychloride, bismuth trioxide, or combinations thereof.

8. The implantable radiopaque stent of any of claims 1-4 or the method of any of claims 5-7, wherein the plurality of radiopaque filaments includes an outer member concentrically disposed about a central core.

9. The implantable radiopaque stent or the method of claim 8, wherein the outer member is formed of nitinol and the central core includes tantalum.

## Patentansprüche

1. Implantierbarer röntgendichter Stent (10; 22), der aufweist:
mehrere röntgendichte Filamente (18; 24; 26; 36; 38; 42), die für eine permanente Anbringung an einer Hohlröhrenstruktur in einer linearen Richtung quer zu einer Länge in einer Längsrichtung (34) der Hohlröhrenstruktur angeordnet sind, wobei die Röhrenstruktur eine röhrenförmige Wand hat, die eine Innenfläche und eine Außenfläche und ein erstes offenes Ende (14) und zweites offenes Ende (12) einander gegenüberliegend definiert, wobei die mehreren röntgendichten Filamente (18; 24; 26; 36; 38; 42) eine externe Bildgebung der Röhrenstruktur auf einem Durchleuchtungs- oder Röntgen-Bildgebungsgerät verbessern,
wobei die Röhrenstruktur mit einem Polymermaterial (50) überzogen ist,
wobei die Filamente (18; 24, 26; 36; 38; 42) an dem zweiten Ende (12) enden, und
wobei die Filamente (18; 24, 26; 36; 38; 42) am ersten Ende (14) in einer Reihe geschlossener Schleifen (15) angeordnet sind, wobei eine jede Schleife (15) einen durch eine Biegung in einem der Filamente (18; 24, 26; 36; 38; 42) definierten Scheitelpunkt hat, und eine gegenüberliegende Basis hat, die durch Überkreuzen von benachbarten Filamente definiert ist, und wobei weiterhin die Scheitelpunkte benachbarter geschlossener Schleifen (15) in Längsrichtung zueinander versetzt sind,
**dadurch gekennzeichnet, dass**
das Polymermaterial (50) weiterhin röntgendichte Partikel aufweist, und der Stent weiterhin nicht-röntgendichte Filamente aufweist, und die röntgendichten Filamente alle in der gleichen linearen Richtung angeordnet sind.

2. Implantierbarer röntgendichter Stent nach Anspruch 1, wobei die mehreren röntgendichten Filamente ein röntgendichtes Material und ein Polymermaterial aufweisen, wobei das röntgendichte Material ein röntgendichtes Pulver ist.

3. Implantierbarer röntgendichter Stent nach Anspruch 1, wobei die mehreren röntgendichten Filamente ein röntgendichtes Material und ein bioabsorbierbares Material aufweisen.

4. Implantierbarer röntgendichter Stent nach Anspruch 1, wobei die Polymerverkleidung biologisch abbaubar ist.

5. Verfahren zum Herstellen eines implantierbaren Stents (10; 22), das aufweist:
Bereitstellen mehrerer Filamente (18; 24, 26; 36; 38; 42), die sowohl röntgendichte wie auch nicht-röntgendichte Filamente aufweisen; und
Anordnen der mehreren Filamente (18; 24, 26; 36; 38; 42) für eine permanente Anbringung an einer Hohlröhrenstruktur in einer linearen Richtung quer zu einer Länge in einer Längsrichtung (34) der Röhrenstruktur, wobei die röntgendichten Filamente alle in der gleichen linearen Richtung angeordnet sind, wobei die Röhrenstruktur eine röhrenförmige Wand bereitstellt, die eine Innenfläche und eine Außenfläche definiert und ein offenes erstes Ende (14) und offenes zweites Ende (12) einander gegenüberliegend hat,
Ausbilden einer Verkleidung für die Röhrenstruktur durch Verkleiden der Röhrenstruktur mit einem Polymermaterial (50), wobei das Polymermaterial röntgendichte Partikel aufweist,
Abschließen der Filamente an dem zweiten Ende (12);
Anordnen der Filamente an dem ersten Ende (14) in einer Reihe geschlossener Schleifen (15), wobei eine jede Schleife (15) einen Scheitelpunkt hat, der eine Biegung in einem der Filamente definiert, und eine gegenüberliegende Basis hat, die durch Überkreuzen von benachbarten Filamenten definiert ist; und
Versetzen der Scheitelpunkte benachbarter geschlossener Schleifen (15) in der Längsrichtung zueinander.

6. Verfahren nach Anspruch 5,
aufweisend das Ausbilden der mehreren röntgendichten Filamente aus einem röntgendichten Material und einem biokompatiblen Material,
aufweisend das Ausbilden der mehreren röntgendichten Filamente aus einem Polymer oder Copolymer, weiterhin aufweisend das Mischen eines röntgendichten Pulvers in einem Siliziumbad, sodass die Verkleidung die röntgendichten Partikel enthält.

7. Implantierbarer röntgendichter Stent nach Anspruch 2 oder 3 oder das Verfahren nach Anspruch 6, wobei das röntgendichte Material ausgewählt ist aus der Gruppe bestehend aus Gold, Platin, Wolfram, Platin-Wolfram, Palladium, Iridium, Platin-Iridium, Rhodium, Tantal, Bariumsulfat, Bismutsubcarbonat, Bismutchloridoxid, Bismut-(III)oxid oder Kombinationen davon.

8. Implantierbarer röntgendichter Stent nach einem der Ansprüche 1 bis 4 oder das Verfahren nach einem der Ansprüche 5 bis 7, wobei die mehreren röntgendichte Filamente ein äußeres Element aufweist, das konzentrisch um einen zentralen Kern angeordnet ist.

9. Implantierbarer röntgendichter Stent oder das Verfahren nach Anspruch 8, wobei das äußere Element aus Nitinol gebildet ist und der zentrale Kern Tantal aufweist.

## Revendications

1. Stent radio-opaque implantable (10 ; 22) comprenant:
une pluralité de filaments radio-opaques (18 ; 24, 26 ; 36 ; 38 ; 42) disposés pour la fixation permanente à une structure tubulaire creuse dans une direction linéaire transversale à une longueur longitudinale (34) de la structure tubulaire creuse, la structure tubulaire ayant une paroi tubulaire qui définit une surface interne et une surface externe et une première extrémité ouverte (14) et une seconde extrémité ouverte (12) opposées, où la pluralité de filaments radio-opaques (18 ; 24, 26 ; 36 ; 38 ; 42) améliore l'imagerie externe de la structure tubulaire sur un appareillage d'imagerie à fluoroscope ou à rayons X,
où la structure tubulaire est revêtue d'un matériau polymérique (50),
où les filaments (18 ; 24, 26 ; 36 ; 38 ; 42) se terminent à la seconde extrémité (12), et
où les filaments (18 ; 24, 26 ; 36 ; 38 ; 42) à la première extrémité (14) sont disposés en une série de boucles fermées (15), chaque boucle (15) ayant un sommet défini par une courbure dans l'un des filaments (18 ; 24, 26 ; 36 ; 38 ; 42) et ayant une base opposée définie par croisement de filaments adjacents, et en outre où les sommets de boucles fermées (15) adjacentes sont décalés longitudinalement les uns des autres,
**caractérisé en ce que**
le matériau polymérique (50) inclut en outre des particules radio-opaques, le stent comprend en outre des filaments non radio-opaques, et les filaments radio-opaques sont tous disposés dans la même direction linéaire.

2. Stent radio-opaque implantable selon la revendication 1, où la pluralité de filaments radio-opaques comprend un matériau radio-opaque et un matériau polymérique, où le matériau radio-opaque est une poudre radio-opaque.

3. Stent radio-opaque implantable selon la revendication 1, où la pluralité de filaments radio-opaques comprend un matériau radio-opaque et un matériau bioabsorbable.

4. Stent radio-opaque implantable selon la revendication 1, où le revêtement polymérique est biodégradable.

5. Procédé pour produire un stent implantable (10 ; 22) comprenant:
la fourniture d'une pluralité de filaments (18 ; 24, 26 ; 36 ; 38 ; 42) comprenant des filaments radio-opaques et des filaments non radio-opaques ; et
la disposition de la pluralité de filaments (18 ; 24, 26 ; 36 ; 38 ; 42) pour la fixation permanente à une structure tubulaire creuse dans une direction linéaire transversale à une longueur longitudinale (34) de la structure tubulaire, où les filaments radio-opaques sont tous disposés dans la même direction linéaire, la structure tubulaire formant une paroi tubulaire définissant une surface intérieure et une surface extérieure et ayant une première extrémité ouverte (14) et une seconde extrémité ouverte (12) opposées,
la formation d'un revêtement pour la structure tubulaire par revêtement de la structure tubulaire avec un matériau polymérique (50), où le matériau polymérique inclut des particules radio-opaques,
la terminaison des filaments à la seconde extrémité (12) ;
la disposition des filaments à la première extrémité (14) en une série de boucles fermées (15), chaque boucle (15) ayant un sommet définissant une courbure dans l'un des filaments et ayant une base opposée définie par croisement de filaments adjacents ; et
le décalage longitudinalement des sommets de boucles fermées (15) adjacentes les uns des autres.

6. Procédé selon la revendication 5,
comprenant la formation de la pluralité de filaments radio-opaques à partir d'un matériau radio-opaque et d'un matériau biocompatible,
comprenant la formation de la pluralité de filaments radio-opaques à partir d'un polymère ou d'un copolymère, comprenant en outre le mélange d'une poudre radio-opaque dans un bain de silicium de sorte que le revêtement inclut lesdites particules radio-opaques.

7. Stent radio-opaque implantable selon la revendication 2 ou 3 ou procédé selon la revendication 6, où le matériau radio-opaque est choisi dans le groupe consistant en l'or, le platine, le tungstène, le platine-tungstène, le palladium, l'iridium, le platine-iridium, le rhodium, le tantale, le sulfate de baryum, le sous-carbonate de bismuth, l'oxychlorure de bismuth, le trioxyde de bismuth, ou leurs combinaisons.

8. Stent radio-opaque implantable selon l'une quelconque des revendications 1-4 ou procédé selon l'une quelconque des revendications 5-7, où la pluralité de filaments radio-opaques inclut un membre externe disposé de manière concentrique autour d'un noyau central.

9. Stent radio-opaque implantable ou procédé selon la revendication 8, où le membre externe est formé de nitinol et le noyau central inclut du tantale.
